# EUROPEAN PATENT SPECIFICATION

(11) **EP 1 424 917 B1**
(45) Date of publication and mention of the grant of the patent: **29.08.2007**
(21) Application number: 02779221.7
(22) Date of filing: 13.09.2002
(51) Int. Cl.: A45C 11/00, A01M 3/00, A45D 40/00

(54) **DEVICE FOR REMOVING A TICK**
VORRICHTUNG ZUM ENTFERNEN EINER ZECKE
dISPOSOTIF POUR ENLEVER UNE TIQUE

(30) Priority: 13.09.2001 DK 200101333; 06.03.2002 DK 200200340
(43) Date of publication of application: 09.06.2004
(73) Proprietor: Kirkegaard, Per, 8660 Skanderborg (DK); Safecard ApS, 8660 Skanderborg (DK)
(72) Inventor: KIRKEGAARD, Per, DK-8660 Skanderborg (DK)
(74) Representative: Schouboe, Anne
(86) International application number: PCT/DK2002/000598
(87) International publication number: WO 2003/022094

(56) References cited:
- DE-A1- 2 717 128
- DE-U- 29 722 310
- US-A- 3 534 887
- US-A- 4 220 244
- US-A- 4 301 923
- US-A- 4 900 663
- US-A- 4 938 764
- US-A- 4 946 033
- US-A- 4 976 718
- US-A- 5 002 323
- US-A- 5 246 449
- US-A- 5 447 511
- US-A- 5 595 569
- US-A- 5 876 409

## Description

The present invention concerns a device, e.g. for personal hygiene, and of the kind indicated in the preamble of claim 1.

US 5,595,569 describes a device for removing a tick or similar insect that has bitten onto and bored down into the skin of a person or an animal. The device is a spoon-shaped device. The outer or leading edge of the spoon bowl has a beveled V-shaped notch of a size to permit the bowl to be passed between the tick and the skin of the host until the scoop engages the tick underneath its body on three sides. A further forward sliding motion of the notched area along the skin releases the tick entirely.

US 5,447,511 describes a tool for removing anchored ticks from the skin of an animal or a human. The tick removal tool includes an elongated member having a longitudinal axis, a lateral axis, a forward edge and a handle end. A tapered slot is formed within the elongated member. The tapered slot is located along the longitudinal axis and diverges towards and merges with the forward edge. The tool is manipulated so that the mouth parts and head of the tick that are embedded in the skin of the animal or human are positioned and secured within the tapered slot.

Furthermore, arrangements are known e.g. as small disposable packings, most often in the form of tear-open bags of plastic or metal film containing a folded wet tissue for cleaning hands and/or face.

On this background, it is the purpose of the invention to provide a new and improved device of the kind mentioned in the preamble, and which in a simple way makes it possible for persons to remove a tick or similar insect that has bitten onto and bored down into the skin of a person or an animal. It may be seen as a further object of the invention to provide a new and improved device of the kind mentioned in the preamble, and which in a simple way makes it possible for persons to carry along one or more devices with the intention of cleaning hands or other body parts when removing the tick or similar insect.

The device according to the invention is characterised in that that said card is made of a relatively stiff material, such as cardboard or plastic, and that said card has a corner area being designed with a slit, for use in removing a tick or corresponding blood sucking insect, which has bitten on to and bored its snout down into the skin of a person or an animal.

The device is designed as a credit card in size, and the device may have a display surface on one side intended for commercial utilisation, and that the device on the other side has one or more hygienic articles which externally are covered by means of one or more film pieces consisting of preferably easily removable (peelable) plastic or metal film. In a simple way is hereby achieved a new and improved device enabling persons to carry with them one or more of said devices with the purpose of cleaning hands or other body parts. The device according to the invention may be manufactured and distributed very cheaply as the device may be partly financed by commercial advertisements due to the display surface.

Suitably, the device according to the invention is thus designed that the hygienic articles are constituted by a tissue (wet tissue) prepared with a cleaning liquid.

With the purpose of facilitating to discern between the individual parts of the device, this may suitably be designed so that the hygienic articles are covered by means of films with mutually different colour or other marking.

Suitably, the device is further designed so that the cover films are provided with a corner or tongue section, which is not connected with the device, and which is intended for use in pulling off the cover films.

The device according to the invention is so designed that the card is made of a relatively stiff material, e.g. cardboard or plastic, that the card e.g. in a corner area is made with a mainly acutely angled slit or cut adapted for use in releasing a tick or similar insect, which has bitten on to and bored its snout down into the skin of a person or an animal. The card may, as a further alternative, on the opposite side preferably have two film covered fields, namely a field in which is laid a disinfecting tissue and a field in which is laid a plaster.

The invention is explained in the following in more detail in connection with the drawing, on which:
- Fig. 1: shows the front side of an embodiment of a device according to the invention in the shape of a "BorreliaCard" for use in releasing a tick,
- Fig. 2: shows the back side of the "BorreliaCard" shown in Fig. 1, and
- Fig. 3: shows a preferred embodiment of a "BorreliaCard" according to the inventtion.

The device according to the invention has a size correspond- ing to a credit card, i.e. designed with rounded corners with a length of about 85 mm, a width of about 54 mm and a total thickness less than 4 mm, preferably less than 2 mm

For example, the device according to the invention may be thus designed that the folded cleaning tissue has a length/width of about 44 x 44 mm. Alternatively, the folded cleaning tissue may have a length/width of about 56 x 44 mm.

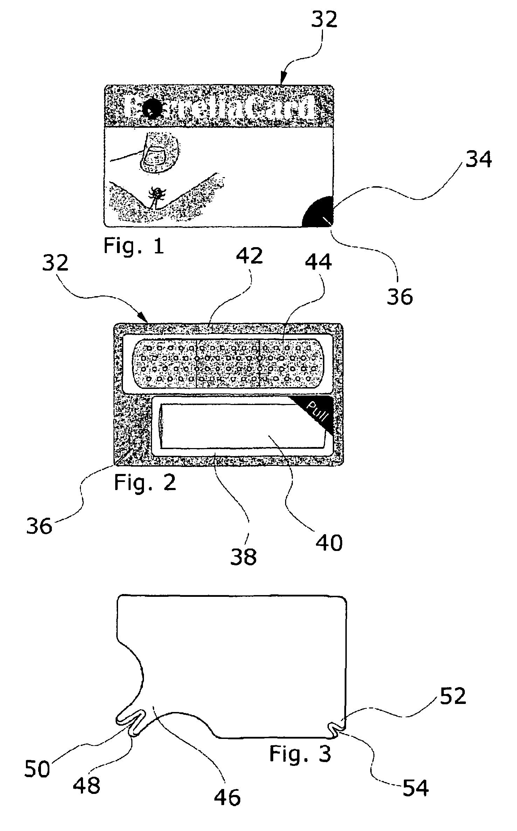

The "BorreliaKort" 32 shown in Figs. 1 and 2 is designed in a corner area 34 with an acutely angled slit or cut-out 36 adapted for use in removing a tick or similar insect that has bitten onto and bored down into the skin of a person or an animal, e.g. a dog.

The card 32 is e.g. printed with the following instructions on the front side:
STOP the TICK!
- Place the slit of the Borrelia card under the tick's abdomen as close to the skin as possible
- Push the card forward so that the tick is lifted out calmly and firmly
- Treat subsequently with chlorhexidine and possibly plaster

The disclosed slit or cut-out 36, which may end up in a circular hole, may alternatively, or as a supplement, also be provided at a short side of the card. The "BorreliaCard" will hereby act as a pre-stressed spring which by itself, after placing under and around the tick, will provide a certain pulling or elevation action on the tick so that it, including mouth parts, will be pulled or lifted off the skin. Cf. another alternative embodiment, the "BorreliaCard" may be designed with a hole with the shape as a keyhole or other suitable profile which makes it possible to move the card down over the tick and to wedge it in a narrowing of the hole.

At the back side of the card 32 (Fig.2) there are two film covered fields, namely a field 38 in which is laid a disinfecting cleaning tissue 40 moistened with chlorhexidine, and a field 42 in which laid a piece of plaster 44.

A preferred embodiment of a "BorreliaCard" is shown in Fig.3 where a corner area is designed with a flexible finger 4 which is designed with a slit or narrowing 50 at an outer end 48 for use in removing a tick or corresponding blood sucking insect. The flexible finger 46 contributes to optimising the function of the "BorreliaCard" so that one gets hold of the tick with certainty. Besides, the "BorreliaCard", cf. Fig.3, is designed in another corner 52 with a supplementing lesser slit or narrowing 54.

The preferred embodiment of a "BorreliaCard" shown in Fig.3 is made of relatively thin plastic material which is preferably without printed instructions, as the "BorreliaCard" in this form is intended to be laid into a folded, card-like brochure or guidance for use which is finally sealed in a transparent plastic packing, which furthermore may contain a number of individually packed cleaning tissues and/or a number of individually packed plasters.

## Claims

1. A device being designed with a slit (36, 54, 50), for use in removing a tick or corresponding blood sucking insect, which has bitten on to and bored its snout down into the skin of a person or an animal, **characterised in that** the device is adapted for keeping in wallet, pocket, bag, or similar place, said device being designed as a credit card in size, that said card is made of a relatively stiff material, such as cardboard or plastic, and that said card has a corner area being designed with the slit (36, 54, 50), for use in removing a tick or corresponding blood sucking insect, which has bitten on to and bored its snout down into the skin of a person or an animal.

2. A device according to claim 1, **characterised in, that** said card, in a corner area (34, 52), is provided with said slit (36, 54), for use in removing a tick or corresponding blood sucking insect.

3. A device according to claim 1, **characterised in, that** said card has a corner area designed with a flexible finger (46), which is provided with said slit (50) at an outer end (48), for use in removing a tick or corresponding blood sucking insect.

4. A device according to any of claims 2-3, **characterised in, that** said card, in one corner area (34, 52), is provided with said slit (36, 54), and that said card has another corner area designed with a flexible finger (46), which is provided with a slit (50) at an outer end (48), for use in removing a tick or corresponding blood sucking insect.

5. A device according to claim 1 **characterised in, that** the card is provided with printing instructions, such as "STOP the TICK", on a front side.

6. A device according to claim 1, **characterised in, that** said device at a back side (32) has two film covered fields, namely a field (38) in which is laid a disinfecting tissue (40) and a field (42) in which is laid a plaster (44).

## Patentansprüche

1. Vorrichtung, die mit einem Schlitz (36, 54, 50) zum Entfernen einer Zecke oder eines entsprechenden blutsaugenden Insekts, das sich in die Haut eines Menschen oder eines Tiers festgebissen oder seinen Rüssel in die Haut eines Menschen oder eines Tiers gebohrt hat, versehen ist, **dadurch gekennzeichnet, dass** die Vorrichtung zur Aufbewahrung in einer Brieftasche, in einer Hosen- oder Jackentasche, in einer Tasche oder an einem ähnlichen Ort geeignet ist, indem die Vorrichtung in ihren Abmessungen wie eine Kreditkarte ausgebildet ist, dass die Karte aus einem relativ steifen Material, wie z.B. Pappe oder Kunststoff, hergestellt ist und dass die Karte einen Eckbereich aufweist, der mit einem Schlitz (36, 54, 50) zum Entfernen einer Zecke oder eines entsprechenden blutsaugenden Insekts, das sich in die Haut eines Menschen oder eines Tiers festgebissen oder seinen Rüssel in die Haut eines Menschen oder eines Tiers gebohrt hat, gestaltet ist.

2. Vorrichtung nach Anspruch 1, **dadurch gekennzeichnet, dass** die Karte in einem Eckbereich (34, 52) mit einem Schlitz (36, 54) zum Entfernen einer Zecke oder eines entsprechenden blutsaugenden Insekts versehen ist.

3. Vorrichtung nach Anspruch 1, **dadurch gekennzeichnet, dass** die Karte einen Eckbereich mit einem flexiblen Finger (46) hat, der mit dem Schlitz (50) an einem äußeren Ende (48) zum Entfernen einer Zecke oder eines entsprechenden blutsaugenden Insekts versehen ist.

4. Vorrichtung nach irgendeinem der Ansprüche 2-3, **dadurch gekennzeichnet, dass** die Karte in einem Eckbereich (34, 52) mit dem Schlitz (36, 54) versehen ist, und dass die Karte einen anderen Eckbereich mit einem flexiblen Finger (46) gestaltet ist, der mit einem Schlitz (50) an einem äußeren Ende (48) zum Entfernen einer Zecke oder eines entsprechenden blutsaugenden Insekts versehen ist.

5. Vorrichtung nach Anspruch 1, **dadurch gekennzeichnet, dass** die Karte mit gedruckten Anweisungen, wie "STOPPT DIE ZECKE", auf einer Vorderseite vorgesehen ist.

6. Vorrichtung nach Anspruch 1, **dadurch gekennzeichnet, dass** die Vorrichtung auf einer Rückseite (32) zwei mit Folie abgedeckten Feldern aufweisen, nämlich ein Feld (38), in das ein Desinfektionstuch (40) gelegt ist, und ein Feld (42), in das ein Pflaster (44) gelegt ist.

## Revendications

1. Dispositif conçu avec une fente (36, 54, 50) pour l'utilisation dans l'enlèvement d'une tique ou d'un autre insecte sanguisorbe qui a piqué et a planté son rostre dans la peau d'une personne ou d'un animal, **caractérisé en ce que** le dispositif est adapté pour être gardé dans un portefeuille, une poche, un sac, ou dans un endroit similaire, le dispositif étant conçu à une taille comme une carte de crédit, **en ce que** ladite carte est fabriquée en un matériau relativement rigide, comme par exemple le carton ou la plastique, et en ce ladite carte présente une zone de coin étant conçu avec la fente (36, 54, 50), pour l'utilisation dans l'enlèvement d'une tique ou d'un autre insecte sanguisorbe qui a piqué et a planté son rostre dans la peau d'une personne ou d'un animal.

2. Dispositif selon la revendication 1, **caractérisé en ce que** ladite carte, dans une zone de coin (34, 52) est muni de ladite fente (36, 54) pour l'utilisation dans l'enlèvement d'une tique ou d'un insecte sanguisorbe correspondant.

3. Dispositif selon la revendication 1, **caractérisé en ce que** ladite carte présente une zone de coin conçu avec un doigt flexible (46) muni de ladite fente (50) dans un bout extrême (48), pour l'utilisation dans l'enlèvement d'une tique ou d'un insecte sanguisorbe correspondant.

4. Dispositif selon une quelconque des revendications 2-3, **caractérisé en ce que** ladite carte, dans une zone de coin (34, 52) est muni de ladite fente (36, 54), et **en ce que** ladite carte présente une autre zone de coin conçu avec un doigt flexible (46) muni d'une fente (50) dans un bout extrême (48) pour l'utilisation dans l'enlèvement d'une tique ou d'un insecte sanguisorbe correspondant.

5. Dispositif selon la revendication 1, **caractérisé en ce que** la carte est muni d'instructions imprimées, comme par exemple "ARRETEZ la TIQUE" sur sa face antérieure.

6. Dispositif selon la revendication 1, **caractérisé en ce que** ledit dispositif sur sa face postérieure (32) présente deux carreaux recouverts en film: un carreau (38) dans lequel est placé un tissu désinfectant (40) et un carreau (42) dans lequel est placé un sparadrap (44).
